# EUROPEAN PATENT APPLICATION

(11) **EP 4 663 090 A1**
(43) Date of publication of application: **17.12.2025**
(21) Application number: 24181545.5
(22) Date of filing: 11.06.2024
(51) Int. Cl.: A61B 1/00, A61B 1/267, A61B 1/273, A61M 16/04

(54) **A DEVICE FOR SUPPORTING PROBE AND/OR ENDOSCOPE PARTS OF MEDICAL DEVICES INTRODUCED THROUGH THE MOUTH OF A PATIENT**

(71) Applicant: Echovice ApS, 5230 Odense M (DK)
(72) Inventor: OLESEN, Jeppe, 5000 Odense C (DK); CHRISTIAN, Ajithan, 5200 Odense V (DK); LAURSEN, Kristian Bach, 5230 Odense M (DK)
(74) Representative: Larsen & Birkeholm A/S

(57) **Abstract**

The present invention relates to a device for supporting probe and/or endoscope parts of medical devices introduced through the mouth of a patient.

## Description

### Technical field of the invention

The present invention relates to medical probes or endoscopes, and more specifically to supporting equipment for use with such medical devices.

### Background of the Invention

Many different medical operations and examinations are performed with probes, such as ultrasonic probes, and endoscopes, such as bronchoscopes or laryngoscopes, introduced through the mouth of a patient. The procedure itself is often highly uncomfortable for the subject. For this reason, it is very common for the subject to be sedated during the procedure. The sedative helps the subject to tolerate the procedure, rather than to oppose it, thus enabling the medical staff to perform the procedure undisturbed. Despite this, the subject will often resist entry of the endoscope by not swallowing as requested and by tensing his or her muscles. Furthermore, involuntary muscular reactions also resist the precise positioning of the probe or endoscope.

As an example, the treatment of leaky heart valves with clips or similar systems depends on the ultrasound images provided. The success of the treatment depends on the ultrasound operator being able to create the best images in the right quality at the right time during the operation. Here, the medical staff are challenged because the esophagus cannot be completely anesthetized during the treatment, even under general anesthesia. The result is that the muscles constantly displace the tube of the ultrasound probe within the esophagus. The ultrasound operators must manually counteract these forces by pushing on the ultrasound probe while controlling the position of the ultrasound probe's head.

In addition, the ultrasound operator must also control the ultrasound apparatus to which the probe is connected, including manipulation of the images shown on the apparatus' monitor. Hence, there is a need to reduce the ultrasound operator's manual tasks.

### Description of the invention

Thus, it is an object of the present invention to provide a simple solution that solves the above-mentioned problems.

The present invention provides a new way of supporting probe and/or endoscope parts of medical devices extending into the mouth of a patient. It is a simple device that makes it possible for one person to handle the probe or endoscope without having to interrupt their work to adjust and reposition the tip of the probe or endoscope. Thereby, the total operating or surveying time can be reduced also reducing the risk of the operation or survey. The device will provide fewer interruptions, less frustration, and better ergonomics to the operator.

A first aspect relates to a device adapted for supporting probe and/or endoscope parts of medical devices introduced through the mouth of a patient, said device comprising:
- a first channel part adapted for fitting into the lumen of a bite block for probe and endoscope equipment or configured as a bite block for probe and endoscope equipment;
- a second channel part with a first wall section adapted for moving between a first position and a second position; and
- a mechanism adapted for displacing said first wall section between its first position and second position;
wherein the first channel part and the second channel part are arranged in extension of each other and together define a common first channel adapted for supporting probe and/or endoscope parts of medical devices introduced through the mouth of a patient.

A bite block is a, often single-use, medical device used to keep the mouth open during invasive imaging procedures or endoscopy to prevent the patient from biting the probe or endoscope. Bite blocks are generally characterized by the following structural features. A tubular body whose internal lumen serves as the channel for passage of the probe or endoscope, and whose top and bottom outer surfaces serve as the surfaces upon which the subject's teeth bite. These surfaces are generally flattened. A wall centrally connected to the outer end of the tubular body, shaped such that it should lie comfortably outside and around the subject's mouth. It is this wall that fulfils the double function of providing a general alignment direction to the tubular body and of preventing the bite block from falling into the mouth. The wall is also known as the front plate. Optionally, a band connected to the bite block and used to strap the bite block firmly to the subject. Some bite blocks are made of a relatively softer plastic material than others, thereby making a better fit within the oral cavity, such that a strap or band is superfluous.

Preferably, when the first wall section is in its second position, the second channel part is adapted to prevent a probe and/or endoscope part from being moved through the first channel, preferably while still allowing for rotational motion of said probe and/or endoscope part about a longitudinal axis of said probe and/or endoscope part.

Preferably, when the first wall section is in its second position, the first channel part defines a second channel arranged parallel to the first channel.

Preferably, the first channel part is configured to define a second channel arranged parallel to the first channel.

A separate access channel, i.e., the second channel, is important for situations where tools need to be introduced into the oral cavity of a patient during anesthesia. The primary tool is the laryngoscope. This instrument, consisting of a handle and a blade, is inserted into the mouth to provide a clear view of the vocal cords by lifting the epiglottis. This visualization is essential for the accurate placement of the endotracheal tube. The endotracheal tube is a flexible tube inserted through the mouth into the trachea, maintaining an open airway and allowing for mechanical ventilation. The tube's cuff can be inflated to seal the trachea and prevent air leaks.

Oropharyngeal airways are also frequently used. These curved devices are inserted into the mouth to keep the tongue from obstructing the pharynx, thereby maintaining airway patency, especially in unconscious patients.

Finally, suction devices may be needed for clearing the oral cavity of secretions, blood, or other fluids that could obstruct the airway.

The device according to the present invention comprises a first channel part either adapted for fitting into the lumen of a bite block for probe and endoscope equipment or configured as a bite block for probe and endoscope equipment. In the latter embodiment, the first channel part may be releasably attached to the device, such as to a second channel part, or a support plate holding the second channel part. The first channel part may thus be for one-time use, i.e., disposable.

A second channel part is provided with a wall section adapted for moving between a first position to a second position. This function allows the user to fix probe and/or endoscope parts of medical devices extending through the second channel part and into the mouth of a patient, thereby preventing it from being displaced. The first and second channel parts are connected, e.g., via a support plate for holding other components of the device, such as the mechanism adapted for displacing the second channel part's wall section between its first retracted position and second extended position. Alternatively, the first and second channel parts are made/formed in one piece, e.g., molded as one piece in a polymeric material.

In one or more embodiments, the first channel part is configured for fitting into the cavity of a bite block for probe and endoscope equipment. The first channel part may comprise two separate insert parts configured for fitting into the cavity of a bite block for probe and endoscope equipment. Alternatively, the first channel part may comprise one or more slits, thereby allowing for press fit assembly with the cavity of a bite block.

The device features an integrated channel component, i.e., the first wall section, designed to facilitate the controlled constriction of the first channel's internal passage. The constriction mechanism preferably comprises a pull wire mechanism, actuated by an actuation unit. In one configuration, the first wall section is configured as a lever arm operably connected to the wire, preferably incorporating a spring element, providing resiliency. Alternatively, the first wall section is made of an elastically deformable material with the wire attached directly to the tip of the wall, rather than to the end of a lever arm.

The inside of the first channel, such as a part of the first wall section, may feature small protrusions, or ribs, to provide better grip on a tool inserted into the first channel. When the actuation unit engages, the user pulls the wire, either causing the lever-like first wall section to pivot and apply a compressive force or directly deforming the elastically flexible first wall section, thereby reducing the first channel's diameter. The spring element or the elastic deformability of the first wall section ensures that the first channel can return to its original shape when the tension is released, enabling precise modulation of the first channel's cross-sectional area for variable control.

In one or more embodiments, the mechanism adapted for displacing said first wall section between its first position and second position comprises a pull wire mechanism. Preferably, the pull wire mechanism comprises a wire with a first end and a second end, wherein the first end of the wire is mounted to the first wall section and wherein the second end is mounted to an actuator.

In one or more embodiments, the actuator comprises a lever arm hingedly mounted to a mounting bracket.

In one or more embodiments, the actuator comprises a pull rod mounted to a guide block, which moves linearly within a guide housing.

In one or more embodiments, the first channel part and the second channel part share a common second wall section. This configuration allows for more stability in the construct.

In one or more embodiments, the mechanism adapted for displacing the first wall section between its first position and second position comprises a spring element adapted for providing resiliency to the first wall section.

In one or more embodiments, the first wall section is configured as a lever arm operably connected to the wire.

In one or more embodiments, the second channel part further comprises a second wall section attached to (e.g., directly, or indirectly via a hinge) a first end of the first wall section, wherein the first wall section comprises a second opposite free end, and wherein the wire is attached to the second opposite free end of the first wall section. Preferably, the first wall section is made of an elastically deformable material, such as a polymeric material.

It may be necessary for the operator of the probe or endoscope to rotate or turn the probe or endoscope around its longitudinal axis, e.g., to obtain a better position for their tip. To be able to rotate (around its longitudinal axis) and still fix (in the longitudinal direction) the probe and/or endoscope parts extending through the second channel part and into the mouth of a patient, the first channel may further comprise one or more ridges, rollers, or ball bearings formed in the second channel part.

A second aspect relates to a medical device comprising a device according to the present invention.

It should be noted that embodiments and features described in the context of one of the aspects of the present invention also apply to the other aspects of the invention.

### Brief description of the figures

Figure 1 show perspective views of a device according to a first embodiment of the present invention, in an open configuration and a closed configuration, respectively.
Figure 2 shows top views of a device according to a first embodiment of the present invention, in an open configuration and a closed configuration, respectively.
Figure 3 show perspective views of a device according to a second embodiment of the present invention, in an open configuration and a closed configuration, respectively.
Figure 4 show top views of a device according to a second embodiment of the present invention, in an open configuration and a closed configuration, respectively.

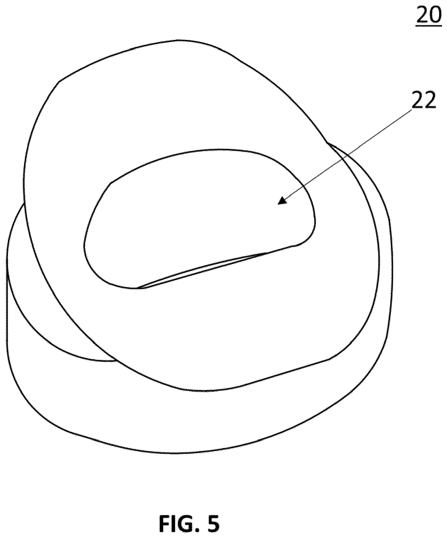

Figure 5 shows a perspective view of a bite block.
Figure 6 show perspective views of an actuator according to a third embodiment of the present invention, in an open configuration and a closed configuration, respectively.
Figure 7 show perspective views of an actuator according to a fourth embodiment of the present invention, in an open configuration and a closed configuration, respectively.

### Detailed description of the invention

The following examples are not meant to be limiting for the scope of the invention but are merely present to show possible and preferred embodiments of the present invention.

In the present context, the term "in general" when used when mentioning a feature relating to the present invention, it must be understood that the feature may be used with all embodiments of the invention, even if the mentioning is made in the detailed part of the document.

In general, the device 100 is adapted to provide support for probe and/or endoscope parts of medical devices extending into the mouth of a patient. The device, in general, is configured with a first channel 130 for the above-mentioned purpose, and a second channel 140 arranged parallel to the first channel 130, e.g., adapted for use in situations where a tool, such as a laryngoscope, needs to be introduced into the oral cavity of a patient during anesthesia, at the same time as probes and/or endoscopes are being used.

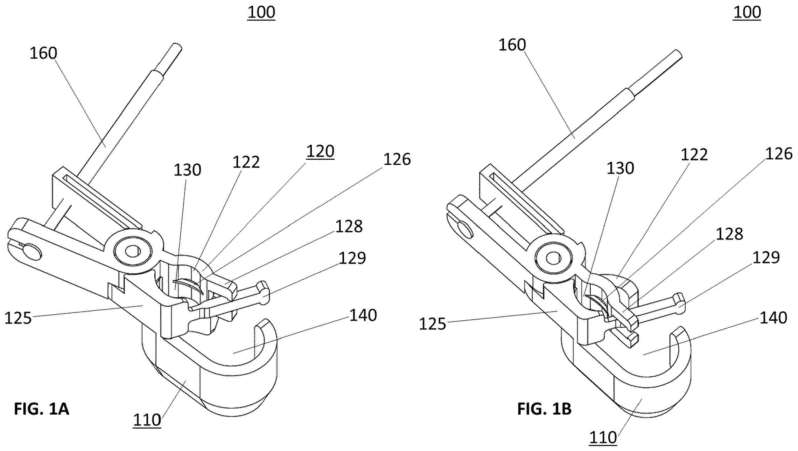

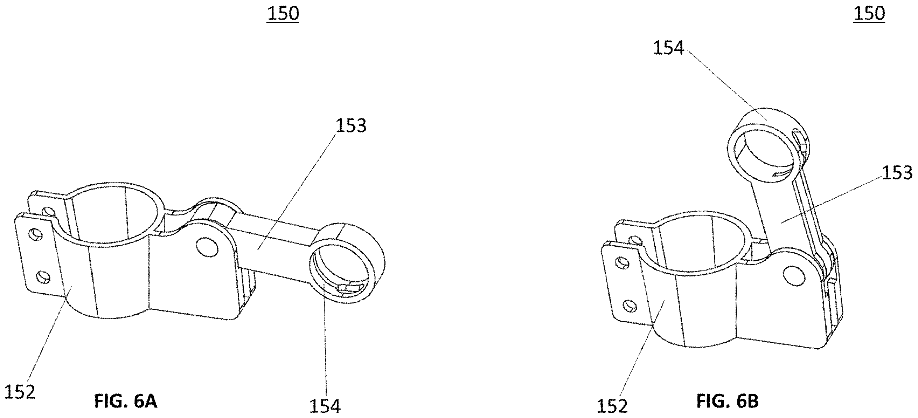

In general, the device 100 (see Figures 1-4) comprises a first channel part 110, and a second channel part 120 that are arranged in extension of each other and together define the first channel 130. The first channel part 110 is here shown adapted for fitting into the lumen 22 of a bite block 20 (see Figure 6) for probe and endoscope equipment but could alternatively be configured as a bite block for probe and endoscope equipment. The second channel part 120 comprises a first wall section 122 adapted for moving between a first position A, and a second position B (here shown in Figure XA, XB, respectively, where X is 1-4).

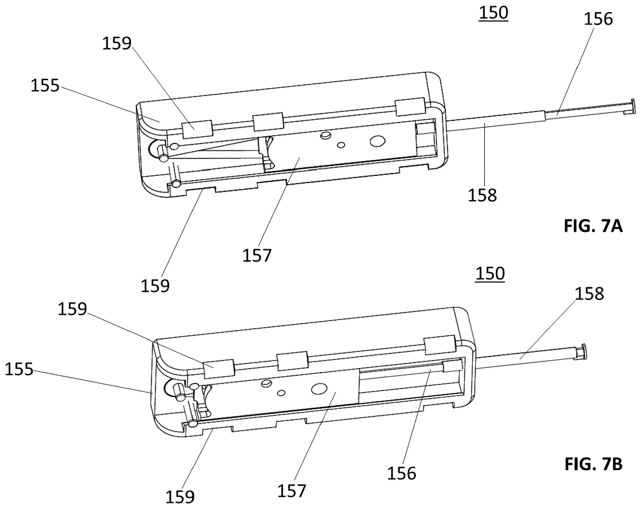

In general, a mechanism is adapted for displacing the first wall section 122 between its first position A and second position B. Here, the mechanism is shown as a pull wire mechanism comprising a wire 160 with a first end and a second end. The first end of the wire 160 is mounted to the first wall section 122 and the second end is mounted to an actuator 150, which is exemplified in two different embodiments in Figures 6 and 7. In general, the second channel part 120, when the first wall section 122 is in its second position B, is adapted to prevent a probe and/or endoscope part from being moved through the first channel 130, preferably while still allowing for rotational motion of the probe and/or endoscope part about a longitudinal axis of said probe and/or endoscope part. The first channel part 110 defines the second channel 140 arranged parallel to the first channel 130.

In general, the pull wire mechanism may comprise a locking mechanism adapted for locking the wire in a specific position, thereby e.g., locking the first wall section 122 in either its first position A or in its second position B, until released/unlocked by the operator.

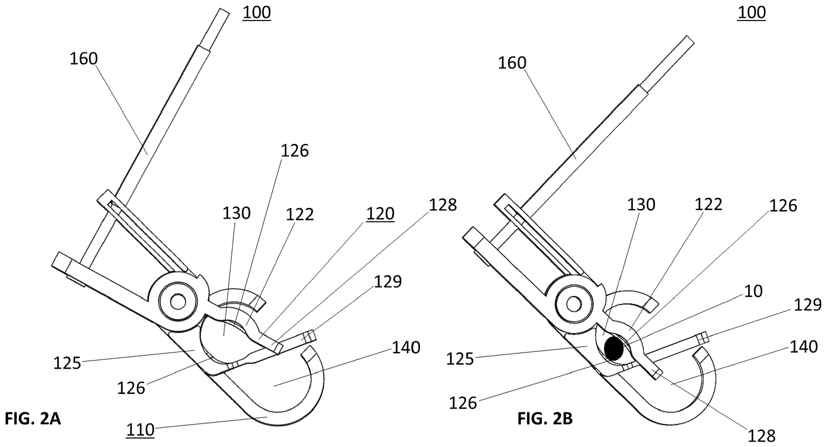

Figures 1 and 2 show a device according to a first embodiment of the present invention, in an open configuration (the first wall section 122 is in its first position A) and a closed configuration (the first wall section 122 is in its second position B), respectively. Here, the first wall section 122 is configured as a lever arm operably connected to the wire 160. This configuration may also be generally applied. The second wall section 125 is shown comprising a guide pin 129, and the first wall section 122 comprises a guide recess 128 adapted for receiving the guide pin 129. This could also be constructed the other way around, such that the first wall section 122 comprises a guide pin, and the second wall section 125 comprises a guide recess. The guide pin 129 and guide recess 128 are together adapted for guiding the first wall section's 122 movement between its first position A and second position B. The mechanism adapted for displacing the first wall section 122 between its first position A and second position B may comprise a spring element (not shown) adapted for providing resiliency to the first wall section 122.

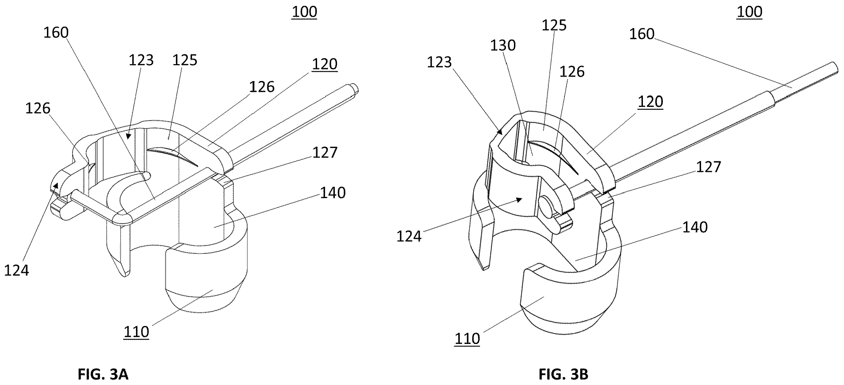

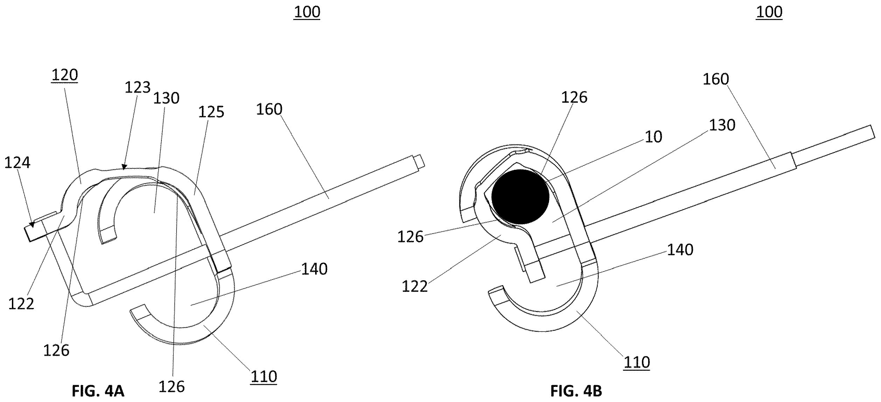

Figures 3 and 4 show a device according to a second embodiment of the present invention, in an open configuration (the first wall section 122 is in its first position A) and a closed configuration (the first wall section 122 is in its second position B), respectively. Here, the second channel part 120 is shown comprising a second wall section 125 attached to a first end 123 of the first wall section 122. The first wall section 122 comprises a second opposite free end 124 attached to the wire 160. The first wall section 122 is made of an elastically deformable material (e.g., a polymeric material) that allows it to bend between its first position A and second position B. The second wall section 125 is also shown comprising a guide recess 127 adapted for supporting and guiding the wire 160.

In general, the first channel 130 is shown comprising ridges 126 formed in the second channel part 120.

Returning to Figure 6, the actuator 150 comprises a lever arm 153 hingedly mounted to a mounting bracket 152. The mounting bracket is adapted for being releasably fastened to the probe or endoscope (not shown), or to another structure providing fixation. The lever arm 153 features a ring pull 154 at one end, designed for easy manipulation with a single finger. When the user inserts their finger into the ring pull and pulls, the lever arm 153 pivots around its hinge point on the mounting bracket 152. This pivoting action transforms the linear force applied by the user's finger into rotational motion. The wire 160 is attached with one end to the lever arm 153, more precisely to the ring pull 154 in the shown configuration, while the other end is attached to first wall section 122, as discussed above in more detail.

As the lever arm 153 rotates, it pulls the wire 160, transmitting the force through the wire to the connected first wall section 122. This tension in the wire thereby activates the device 100. Conversely, when the user releases the ring pull, the lever arm returns to its original position, e.g., assisted by a spring (not shown). This action loosens the wire, reducing the tension and consequently causing the device 100 to deactivate, i.e., to loosen its grip on a probe or endoscope part 10.

Thus, through the simple action of pulling and releasing the ring pull 154, the user can exert precise control over the device, achieving the desired operation with minimal effort. The efficiency of this system lies in its ability to amplify a small input force into a significant mechanical action through the principles of leverage and tension.

Returning to Figure 7, the actuator 150 comprises a pull rod 156 mounted to a guide block 157, which moves linearly within a guide housing 155. This setup offers an effective means of operating another mechanism through the controlled tension of a wire. The wire 160 is attached with one end to the guide block 157, while the other end is attached to first wall section 122, as discussed above in more detail.

In this configuration, the guide housing 155 serves as a fixed structure adapted for being mounted to the probe or endoscope (not shown), or to another structure providing fixation. The guide housing 155 ensures that the guide block 157 and pull rod 156 can move in a precisely controlled linear path. The pull rod 156, which is directly attached to the guide block 157, extends out of the guide housing 155, providing a handle or grip for the user. In the disclosed embodiment, a part of the guide housing 155 is configured as a guide tube 158.

When the user pulls on the pull rod 156, the guide block 157 moves linearly within the guide housing 155, sliding along its predefined path. This linear motion of the guide block 157 is crucial because it ensures that the attached wire 160 experiences a consistent and direct pulling force. As the guide block 157 moves, it pulls the wire 160, transmitting the force along the length of the wire 160 to the connected first wall section 122. The pulling action increases the tension in the wire, thereby activating the device 100. The guide housing 155 ensures that this movement is stable and precise, preventing any lateral deviation that could affect the performance of the mechanism.

When the user pushes the pull rod 156 back into the guide housing 155, the guide block 157 moves in the opposite direction, which loosens the wire 160 and reduces the tension. This reverse motion loosens the wire, reducing the tension and consequently causing the device 100 to deactivate, i.e., to loosen its grip on a probe or endoscope part 10. This type of mechanism offers a high degree of control and reliability. The linear motion facilitated by the guide block 157 within the guide housing 155 ensures that the force applied by the user is efficiently converted into the desired mechanical action, with minimal loss of energy and maximum precision.

### References

- 10: Probe and/or endoscope part
- 20: Bite block
- 22: Lumen
- 100: Device
- 110: First channel part
- 120: Second channel part
- 122: First wall section
- 123: First end
- 124: Second end
- 125: Second wall section
- 126: Ridge
- 127: Guide recess
- 128: Guide recess
- 129: Guide pin
- 130: First channel
- 140: Second channel
- 150: Actuator
- 152: Bracket
- 153: Lever arm
- 154: Ring pull
- 155: Guide housing
- 156: Pull rod
- 157: Guide block
- 158: Guide tube
- 159: Recess
- 160: Wire

## Claims

1. A device (100) adapted for supporting probe and/or endoscope parts (10) of medical devices introduced through the mouth of a patient, said device (100) comprising:
- a first channel part (110) adapted for fitting into the lumen (22) of a bite block (20) for probe and endoscope equipment, or configured as a bite block for probe and endoscope equipment;
- a second channel part (120) with a first wall section (122) adapted for moving between a first position (A) and a second position (B); and
- a mechanism adapted for displacing said first wall section (122) between its first position (A) and second position (B);
wherein the first channel part (110) and the second channel part (120) are arranged in extension of each other and together define a common first channel (130) adapted for supporting probe and/or endoscope parts (10) of medical devices introduced through the mouth of a patient;
wherein the second channel part (120), when said first wall section (122) is in its second position (B), is adapted to prevent a probe and/or endoscope part from being moved through said first channel (130) while still allowing for rotational motion of said probe and/or endoscope part about a longitudinal axis of said probe and/or endoscope part;
**characterized in that** the first channel part (110) is configured to define a second channel (140) arranged parallel to the first channel (130).

2. The device according to claim 1, wherein the mechanism adapted for displacing said first wall section (122) between its first position (A) and second position (B) comprises a pull wire mechanism.

3. The device according to claim 2, wherein said pull wire mechanism comprises a wire with a first end and a second end, wherein the first end of the wire is mounted to the first wall section (122) and wherein the second end is mounted to an actuator (150).

4. The device according to any one of the claims 1-3, wherein the first channel part (110) and the second channel part (120) share a common second wall section (125).

5. The device according to any one of the claims 1-4, wherein the mechanism adapted for displacing said first wall section (122) between its first position (A) and second position (B) comprises a spring element adapted for providing resiliency to the first wall section (122).

6. The device according to any one of the claims 2-5, wherein the first wall section (122) is configured as a lever arm operably connected to the wire (160).

7. The device according to claim 6, wherein the second wall section (125) comprises a guide pin (129), wherein the first wall section (122) comprises a guide channel or guide recess (128) adapted for receiving said guide pin (129), or vice versa, and wherein said guide pin (129) and guide channel or guide recess (128) together are adapted for guiding the first wall section's (122) movement between its first position (A) and second position (B).

8. The device according to any one of the claims 2-5, wherein the second channel part (120) further comprises a second wall section (125) attached to a first end (123) of the first wall section (122), wherein the first wall section (122) comprises a second opposite free end (124), wherein the first wall section (122) is made of an elastically deformable material, and wherein the wire (160) is attached to the second opposite free end (124) of the first wall section (122).

9. The device according to claim 8, wherein the second wall section (125) comprises a guide channel or guide recess (127) adapted for supporting and guiding the wire (160).

10. The device according to any one of the claims 1-9, wherein the first channel (130) comprises one or more ridges (126) formed in the second channel part (120).

11. The device according to any one of the claims 1-10, wherein said first channel part (110) is configured for fitting into the lumen (22) of a bite block (20) for probe and endoscope equipment.

12. The device according to any one of the claims 3-11, wherein the actuator (150) comprises a lever arm (153) hingedly mounted to a mounting bracket (152).

13. The device according to any one of the claims 3-11, wherein the actuator (150) comprises a pull rod (156) mounted to a guide block (157), which moves linearly within a guide housing (155).

14. A medical device comprising a device according to any one of the claims 1-13.
